# EUROPEAN PATENT APPLICATION

(11) **EP 0 692 716 A1**
(43) Date of publication of application: **17.01.1996**
(21) Application number: 94110796.3
(22) Date of filing: 12.07.1994
(51) Int. Cl.: G01N 33/573, C07K 16/40

(54) **Method of assaying for glutathione- S-transferase and diagnostic test therefrom**

(71) Applicant: BAYER AG, D-51368 Leverkusen (DE)
(72) Inventor: Guder, Walter G. Prof.Dr.med., Englschalkinger Str.77 D-81925 München (DE); Hofmann, Walter Dr. med., Englschalkinger Str.77 D-81925 München (DE)

(57) **Abstract**

A method of assaying for glutathione-S-transferase in urine, a method of diagnosing acute kidney damage and a method of monitoring the nephrotoxic effects of a drug are disclosed.

## Description

### FIELD OF THE INVENTION

The present invention relates to the specific binding partner assay of urine for an enzyme, wherein presence of the enzyme is indicative of a diseased or damaged kidney. More particularly, the present invention relates to a specific binding partner method of assaying urine for the enzyme glutathione-S-transferase (GST). The method is useful in a diagnostic test for acute kidney damage, and specifically for acute damage to the proximal tubule of the kidney.

### BACKGROUND OF THE INVENTION

GST is present in several different tissues and plays a central role in the biological transformation of drugs and toxic chemicals. The catalytic action of GST produces glutathione conjugates, which are either excreted or hydrolyzed to cysteine derivatives, and subsequently N-acetylated to yield mercapturic acids. In particular, GST has been identified in the liver, kidney, adrenal gland, duodenum and gonads. The enzyme is present in high concentration, i.e., 5% and 2% respectively, of the cytoplasmic protein content of the liver and proximal tubules of the kidney.

The release of an enzyme (such as GST) into a body fluid during various pathological conditions is known. Therefore, detection of such enzymes assist in the diagnosis of many disorders. For example, the assay for a particular enzymatic protein, or for enzyme activity, in serum and plasma has been used in diagnosing heart and liver diseases. The assay for a particular enzyme in diagnosing kidney damage and diseases is not customary however.

The kidneys are a target organ for the toxic action of various drugs and chemicals. The kidney is therefore subject to either chronic or acute damage. Nephropathy, or a kidney abnormality, due to the toxic action of drugs and chemicals often is manifested as proximal tubular necrosis.

In the kidney, GST is reported to be localized in the proximal tubular epithelium (W.G. Guder et al. "Enzyme Distribution Along the Nephron", Kidney International, Vol. 26, pp. 101-111 (1984)). An increased permeability of renal epithelial cells can lead to release of proteins into the urine. Accordingly, investigators have detected GST in urine (which normally is free of GST) because of leakage from damaged tubular cells.

The proximal tubule is an important component of the kidney because the proximal tubule reabsorbs about 65 to about 70 percent of the sodium ions that pass through the glomerulus. Nephropathy often leads to a decreased glomerular filtration rate which is manifested by a rise in plasma creatinine concentration and a rise in plasma urea concentration, and by a fall in daily urine volume. However, significant kidney damage can occur before increased amounts of creatinine and urea are detectable in the plasma. Accordingly, a sensitive test that reveals kidney damage in the early stages would be desirable.

Various investigators have studied the urinary excretion of enzymes in an effort to diagnose nephropathy of kidney graft rejection. Investigations are reported in:
W.E. Stroo et al., "Enzymes of Renal Origin in Urine as Indicators of Nephrotoxicity" Toxicol. Appl. Pharmacol., 39, pp. 423-434 (1977);
E. Bombard et al., "Urinary Enzyme Measurements as Sensitive Indicators of Chronic Cadmium Nephrotoxicity" Contributions to Nephrology, Vol. 42, pp. 142-147, (1984);
L. Backman et al., "Glutathione Transferase in the Urine: A Marker for Posttransplant Tubular Lesions" Kidney International, 33, pp. 571-577 (1988);
N. Tateoka et al., "Purification and characterization of Glutathione S-Transferase in Human Kidney." Clinica Chemica Acta, 166, pp. 207-218 (1987); and,
P. Kotanko et al., "Urinary Enzyme Analysis in Renal Allograft Transplantation", Clinica Chemica Acta, 160, pp. 137-146 (1986).
The above-listed publications disclose the measurement of GST enzyme activity or the radioimmunoassay of GST in animals and humans after administration of nephrotoxic agents or nephrotoxic drugs.

Other publications describing GST and its presence in the kidney include:
W.G. Guder et al., "Biochemical Characterization of Individual Nephron Segments" Handbook of Physiology, The Kidney (E. Windhager, ed.), Oxford University Press, New York, pp. 2119-2164 (1992);
V.S. Singh et al., "Purification and Characterization of Glutathione-S-Transferase of Human Kidney." Biochem. J., 246, pp. 179-186 (1987); and
W.H. Habig et al., "Glutathione S-Transferase, The First Step in Mercapturic Acid Formation", J.Biol.Chem., 249, pp. 7130-7139 (1974).
Japanese Patent Application JP 90-131046 discloses a method and reagents for the immunoassay of GST in blood.

A need still exists however for a sensitive assay of GST in urine, wherein assay results can be correlated to kidney damage, and particularly to acute damage to the proximal tubule. Such an assay and diagnostic method are important for individuals being treated with aminoglycosides or other drugs that are known to cause kidney damage. By monitoring the amount of GST in urine, an individual then can monitor the effects of a nephrotoxic drug like an aminoglycoside on the kidney, and adjust dosages and treatment schedules accordingly.

### SUMMARY OF THE INVENTION

The present invention is directed to a specific binding partner method of assaying urine for a predetermined protein, and specifically, for a predetermined enzyme. In particular, the present invention is directed to the specific binding partner assay of urine for GST. The presence of GST in urine is an indicator of acute kidney damage, and particularly acute damage of the proximal tubule.

Previously, an increased creatinine concentration in plasma has been used to diagnose kidney damage. However, in the case of an acutely-damaged kidney, GST is present in urine before an increased creatinine concentration in plasma is detectable. Therefore, an assay of urine for GST provides an early detection test for acute kidney damage.

Accordingly, one aspect of the present invention is to provide a method of detecting acute tubular damage to the kidneys before conventional manifestations of kidney damage are apparent. Another aspect of the present invention is to provide a method of assaying urine specifically for GST, and correlating the amount of GST in the urine to acute damage to the proximal tubule of the kidney.

Another aspect of the present invention is to assay urine for GST by a specific binding partner method, as opposed to an enzymatic method. The specific binding partner assay for GST is more sensitive than an enzymatic activity assay. The specific binding partner assay also is independent of enzymatic inhibitors present in urine, thereby providing a more accurate assay for GST.

The above and other aspects and advantages of the present invention will become apparent from the following detailed description of the invention.

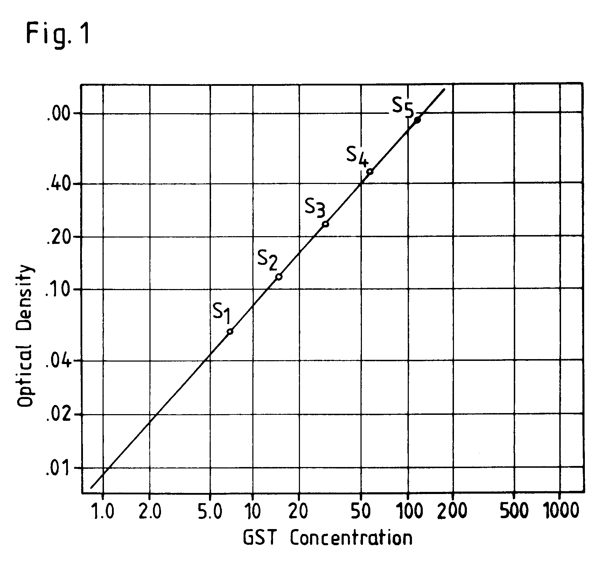

### BRIEF DESCRIPTION OF THE DRAWING

The figure is a plot of GST concentration versus optical density for a series of GST assays using a specific binding partner assay.

### DETAILED DESCRIPTION OF THE PREFERRED EMBODIMENTS

GST is present in several different tissues, including the kidney. Specifically, GST is present in the proximal tubule of the kidney. GST is not present in the urine of a healthy individual, but acute damage to the proximal tubule of the kidney can result in a release of GST into the urine.

For example, various drugs can damage the kidney, and therefore the dosage of such drugs is monitored carefully. The damage from such drugs can be monitored by assaying serum or plasma for creatinine. Typically, creatinine concentration increases with the kidney damage.

The assay for creatinine has disadvantages because although an increased creatinine concentration is an indicator of kidney damage, the assay does not indicate where kidney damage has occurred, or whether the damage is acute or chronic. The present method overcomes these disadvantages, and provides an early detection of acute damage to the proximal tubule of the kidney by the specific binding partner assay of urine for GST.

To illustrate that the presence of GST in urine is an indicator of acute damage to the proximal tubules, the following tests were performed on a reference population and on individuals suffering from various kidney diseases. In each test, the urinary GST concentration was measured using a specific binding partner assay for GST, and more particularly a sandwich immunoassay described in detail hereinafter. First, 116 urine samples from 29 different individuals were assayed for GST. This reference population of healthy individuals had a urinary GST concentration in the range of less than 10 to about 40 µg GST/g creatinine (micrograms of GST per gram of creatinine).

The concentration of GST in urine is expressed as µg GST/g creatinine to make the assay more independent of total urine concentration. The amount of an analyte excreted in urine often is not reported as a simple concentration because the concentration of an analyte varies with the concentration of the urine as a whole. Therefore, the concentration of an analyte is expressed as a ratio of the concentration of the analyte to the concentration of creatinine in the urine sample.

Creatinine is selected as the second analyte in the ratio because creatinine is excreted at an essentially constant rate, which is directly proportional to the muscle mass of an individual. Therefore, a ratio of analyte concentration to creatinine concentration provides a more accurate assay because the analyte assay is more independent of the effects of very dilute or very concentrated urine samples. The concentration of analyte and the concentration of creatinine both are inversely proportional to the amount of water in the urine sample. Since creatinine excretion is essentially constant, the effects of water in the urine sample are negated. The concentration of the analyte therefore is normalized.

Typically, one liter of normal urine includes a one gram of creatinine. Therefore, the reference range of less than about 10 to about 40 µg GST/g creatinine for healthy individuals is essentially equal to a reference range of less than about 10 to about 40 µg GST/L (liter) of urine.

The reference range of less than about 10 to about 40 µg GST/g creatinine, i e., less than about 10 to about 40 µg GST/L urine, was compared to the urinary GST concentration of six individuals being treated with an aminoglycoside (i.e., gentamicin). Gentamicin is known to be nephrotoxic, and individuals treated with gentamicin exhibit an increased urinary GST concentration. At the onset of the gentamicin treatment schedule, two of the six individuals had a GST concentration above 40 µg GST/g creatinine, or above the normal upper limit of the reference range. During the course of the gentamicin treatment, the median urine GST concentration of the six individuals increased to 373 µg GST/g creatinine. The highest GST concentration was 1899 µg GST/g creatinine. The GST concentrations were determined by the present specific binding partner assay for GST.

In contrast to the acute kidney damage caused by gentamicin, thirty-two individuals suffering from chronic glomerulonephritis or a tubulo-interstitial nephropathy exhibited a urinary GST concentration below 40 µg GST/g creatinine. In addition, three individuals having an acute kidney transplant rejection exhibited urinary GST concentrations below 40 µg GST/g creatinine throughout the investigation.

The urinary GST concentration therefore was in the normal range of less than 10 to about 40 µg GST/g creatinine (i.e., less than about 10 to about 40 µg GST/L urine) for healthy individuals, individuals having chronic kidney ailments and individuals having an acute transplant rejection. However, individuals suffering from acute kidney damage (e.g., individuals treated with gentamicin) exhibited up to a 50 fold increase in urinary GST concentration. Therefore, measurement of urinary GST concentration is an indicator of acute damage to the proximal tubule of the kidney, the site in the kidney where GST is located.

The presence of GST in the proximal tubule is known. However, until the method of the present invention, the correlation between an increased urinary GST concentration and acute kidney damage has not been made. In addition, previous methods of assaying for GST measured the enzymatic activity of the test sample, and correlated the enzymatic activity to GST concentration. Often, the enzymatic activity measurements were inaccurate because a portion of the GST in urine can be damaged and therefore is enzymatically inactive. In addition, enzymatic inhibitors can be present in the urine. Accordingly, measuring enzymatic activity yields erroneously low values for urinary GST concentration. However, the present specific binding partner method provides an accurate and sensitive assay for GST in urine because damaged and undamaged GST is assayed by a specific binding partner for urinary GST, and the specific binding partner is unaffected by enzymatic inhibitors.

The specific binding partner assay for GST was performed by first generating a specific binding partner for GST by standard procedures known to those skilled in the art. An exemplary procedure is illustrated "Methods in Enzymatic Analysis", H.U. Bergmeyer (Ed.), Vol. IX, pp. 15-37 (1985).

In general, two rabbits were subcutaneously immunized with a solution of 50 µg (microgram) of GST (the antigen) mixed with 500 µL (microliters) Alugel and Freund's adjuvant. Booster injections of the GST antigen solution were administered to the rabbits once a week for a period of 5 weeks after the first immunization.

More particularly, pure kidney GST (50 µg) was emulsified in Freund's adjuvant (ratio 1:1 v/v) with the addition of 1M sodium chloride solution, as needed, to promote emulsification and provide a final volume of 500 µL. The resulting emulsion was injected into the rabbits, subcutaneously, at multiple sites. Booster injections were performed weekly, for four consecutive weeks, with a solution containing 20 µg GST in 500 µL aqueous sodium chloride. Blood was collected from the rabbits one week following the final (i.e., fifth) injection.

The specific anti-GST serum IgG fraction was isolated by affinity chromatography on a GST CNBr-activated Sepharose 4B affinity column. A GST CNBr-activated Sepharose 4B column was equilibrated overnight with PBS buffer having a pH of 7.2. Then, the rabbit serum (8 mL) was pumped over the CNBr-activated Sepharose 4B column overnight. The column then was washed with PBS buffer until no protein was detected in the eluted fractions. The IgG fraction was eluted using a 0.1M glycine hydrochloride buffer having a pH of 3.0.

A sandwich immunoassay for GST then was developed. The specific antibodies against GST were isolated by affinity chromatography by CNBr-activated Sepharose, which was coupled with pure GST. The isolated anti-GST IgG fraction was labelled with Biotin by the method disclosed in P.V. Subba Rao et al., "An Avidin-Biotin MicroELISA for Rapid Measurement of Total and Allergen Specific Human IgE", J. Immunol.Methods, 57, pp. 71-85 (1983). Concentrations of GST were measured by means of a calibration curve.

The specific binding partner method detected and measured GST over the concentration range of about 6 to about 110 µg GST/L urine (i.e., about 6 to about 100 µg GST/g creatinine). The figure is a plot of GST concentration versus optical density for the assay of urine samples containing 6 µg GST/L through 110 µg GST/L urine. The plot illustrates the excellent linear response between GST concentration and optical density provided by the method of the present invention.

It should be understood however that the present specific binding partner method is useful for assaying urines having a GST concentration outside of the range of about 6 to about 110 µg GST/L of urine sample. The linear response illustrated in the figure extends below about 6 µg GST/L of urine and above about 110 µg GST/L of urine. Furthermore, if necessary and as known to persons skilled in the art, urine samples having a GST concentration above about 110 µg GST/L of urine, and up to about 2x10⁴ µg GST/L of urine, can be diluted with water at a sufficient ratio of urine sample to water, such as about 1:1 to about 1:1000, to provide a test solution having a GST concentration of about 6 to about 110 µg GST/L of urine. Assay results then can be corrected by the dilution factor to provide the true GST concentration of the urine sample. Therefore, a urine sample containing up to 2x10⁴ µg GST/L urine, when properly diluted, can be assayed for GST concentration by the method of the present invention.

Normal urine has a GST concentration of less than about 10 µg/L to about 40 µg/L. Individuals treated with aminoglycosides exhibited increased median urine GST concentration of about 373 µg GST/g creatinine. Therefore, it is estimated that a GST concentration in excess of about 40 µg/L indicates the onset of acute damage to the proximal tubule of the kidney.

The present specific binding partner method also is analytically precise, as demonstrated by the following test data:

| | Interaassay Variation¹⁾ | Interassay Variation²⁾ |
|---|---|---|
| Concentration (µg/L) | 5.1 | 5.1 |
| Measurement(n)³⁾ | 12 | 13 |
| CV(%)⁴⁾ | 4.8 | 11.2 |
| | | |

| | | |
|---|---|---|
| 1) measurements within a series of 12 assays; | | |
| 2) measurements from day to day over a period of eleven days; | | |
| 3) n = number of tests; and | | |
| 4) CV = standard deviation/mean x 100%. | | |

The increased urine GST concentration in an individual suffering from acute kidney damage as opposed to chronic kidney damage indicates that GST is an indicator of acute tubular damage. In addition, the present specific binding partner assay for GST provides a more sensitive assay for acute kidney damage than prior methods that measure serum creatinine levels, and a more accurate assay for GST than prior enzymatic detection methods. In addition, the assay for urinary GST provides an earlier indication of acute tubular damage.

Obviously, many modifications and variations of the invention as hereinbefore set forth can be made without departing from the spirit and scope thereof and therefore only such limitations should be imposed as are indicated by the appended claims.

## Claims

1. A method of assaying a urine sample for glutathione-S-transferase comprising:
(a) incorporating a specific binding partner for glutathione-S-transferase into a device in a manner such that the device is capable of exhibiting a detectable and measurable response to interactions between glutathione-S-transferase and the specific binding partner;
(b) contacting the device with the urine sample;
(c) examining the device for a detectable response; and,
(d) correlating the detectable response in the device to the amount of glutathione-S-transferase in the urine sample.

2. The method of claim 1 wherein the urine sample is undiluted.

3. The method of claim 1 wherein the urine sample is diluted.

4. The method of claim 1 wherein the glutathione-S-transferase is present in the urine sample in an amount of up to about 2x10⁴ µg GST/L of urine.

5. The method of claim 2 wherein glutathione-S-transferase is present in the urine sample in an amount of about 6 to about 110 µg GST/L of urine.

6. The method of claim 3 wherein the glutathione-S-transferase is present in the diluted urine sample in an amount of about 6 to about 110 µg GST/L of urine.

7. A method of diagnosing acute kidney damage comprising:
(a) contacting a urine sample with a device capable of undergoing a detectable and measurable response to glutathione-S-transferase present in the urine sample, said device having incorporated therein a specific binding partner for glutathione-S-transferase;
(b) examining the device for a response; and
(c) correlating the response to the amount of glutathione-S-transferase in the urine sample.

8. The method of claim 7 wherein the glutathione-S-transferase in an amount of above about 40 µg GST/L of urine is indicative of acute kidney damage.

9. The method of claim 7 wherein the acute damage is in a proximal tubule of a kidney.

10. A method of monitoring the nephrotoxic effects of a drug comprising:
(a) contacting a urine sample with a device capable of undergoing a detectable and measurable response to glutathione-S-transferase present in the urine sample, said device having incorporated therein a specific binding partner for glutathione-S-transferase;
(b) examining the device for a response;
(c) correlating the response to the amount of glutathione-S-transferase in the urine sample; and
(d) comparing the amount of glutathione-S-transferase to the amount of glutathione-S-transferase in a previous urine sample, wherein an increased amount of glutathione-S-transferase in the urine is indicative of the nephrotoxic effects of the drug.
